# EUROPEAN PATENT SPECIFICATION

(11) **EP 2 542 547 B1**
(45) Date of publication and mention of the grant of the patent: **24.01.2018**
(21) Application number: 11731488.0
(22) Date of filing: 26.02.2011
(51) Int. Cl.: C07D 339/04, A61K 31/385, A61P 1/16, A61P 35/00, A61P 25/16, A61P 25/28, A61P 39/04

(54) **COMPOUNDS, COMPOSITIONS, FORMULATIONS AND THEIR USES IN THE TREATMENT OF DISEASES RELATED TO COPPER RETENTION OR HEPATIC DISORDERS**
VERBINDUNGEN, ZUSAMMENSETZUNGEN, FORMULIERUNGEN UND IHRE VERWENDUNG BEI DER BEHANDLUNG VON ERKRANKUNGEN IM ZUSAMMENHANG MIT KUPFERRETENTION ODER LEBERERKRANKUNGEN
COMPOSÉS, COMPOSITIONS ET LEUR UTILISATION DANS LE TRAÎTEMENT DE MALADIES LIÉES À LA RETENTION DU CUIVRE OU OU DÉSORDRES HÉPATIQUES

(30) Priority: 05.03.2010 US 310719 P
(43) Date of publication of application: 09.01.2013
(73) Proprietor: Krisani Bioscience (P) Ltd., 500 033 Hyderabad (IN)
(72) Inventor: Kandula, Mahesh, Andhra Pradesh 533434 (IN)
(74) Representative: Elend, Almut Susanne
(86) International application number: PCT/IB2011/000592
(87) International publication number: WO 2011/107881

(56) References cited:
- WO-A1-2009/018152
- WO-A2-2005/107723
- SWARAN J.S. FLORA ET AL: "Chelation in Metal Intoxication", INTERNATIONAL JOURNAL OF ENVIRONMENTAL RESEARCH AND PUBLIC HEALTH, vol. 7, no. 12, 1 January 2010 (2010-01-01), pages 2745-2788, XP55010833, ISSN: 1661-7827, DOI: 10.3390/ijerph7072745
- GREGUS Z ET AL: "Effect of lipoic acid on biliary excretion of glutathione and metals", TOXICOLOGY AND APPLIED PHARMACOLOGY, ACADEMIC PRESS, US, vol. 114, no. 1, 1 May 1992 (1992-05-01), pages 88-96, XP024883585, ISSN: 0041-008X, DOI: 10.1016/0041-008X(92)90100-7 [retrieved on 1992-05-01]
- OU P ET AL: "THIOCTIC (LIPOIC) ACID: A THERAPEUTIC METAL-CHELATING ANTIOXIDANT?", BIOCHEMICAL PHARMACOLOGY, PERGAMON, OXFORD, GB, vol. 50, no. 1, 1 January 1995 (1995-01-01), pages 123-126, XP000607456, ISSN: 0006-2952, DOI: 10.1016/0006-2952(95)00116-H
- BHATT K ET AL: "Oral co-administration of alpha-lipoic acid, quercetin and captopril prevents gallium arsenide toxicity in rats", ENVIRONMENTAL TOXICOLOGY AND PHARMACOLOGY, ELSEVIER, AMSTERDAM, NL, vol. 28, no. 1, 1 July 2009 (2009-07-01), pages 140-146, XP026104362, ISSN: 1382-6689, DOI: 10.1016/J.ETAP.2009.03.012 [retrieved on 2009-04-07]
- KOBAYASHI S ET AL: "Combination treatment with penicillamine and trientine in a patient with Wilson's disease", PEDIATRICS INTERNATIONAL, vol. 47, no. 5, 1 October 2005 (2005-10-01), pages 589-591, XP002503583, ISSN: 1328-8067, DOI: 10.1111/J.1442-200X.2005.02106.X
- Ronald F. Pfeiffer: "Wilson's Disease", , 1 April 2007 (2007-04-01), pages 123-132, XP55011085, DOI: 10.1055/s-2007-971173 Retrieved from the Internet: URL:http://www.thieme.com/index.php?page=s hop.product_details&flypage=flypage.tpl&pr oduct_id=916&category_id=90&option=com_vir tuemart&Itemid=53 [retrieved on 2011-11-03]

## Description

### TECHNICAL FIELD

This disclosure relates to a compound of formula 1A and its use in treating hepatic disorders, for example Wilson's disease.

### BACKGROUND ART

Metal toxicity may occur due to essential metal overload or exposure to heavy metals from various sources. Most metals are capable of forming covalent bonds with carbon, resulting in metal-organic compounds. Metals and metal compounds interfere with functions of various organ systems like the central nervous system (CNS), the haematopoietic system, liver, kidneys, etc. (Flora et al.2010).

Metal accumulation has been responsible for many dysfunctions in liver diseases. Pathophysiologic mechanisms responsible for cerebral dysfunction and neuronal cell death in hepatocerebral disorders, such as Wilson's Disease, post-shunt myelopathy, hepatic encephalopathy, and acquired non-Wilsonian hepatocerebral degeneration are a major feature of hepatocerebral disorders. Morphologic changes to astrocytes (Alzheimer type II astrocytosis) include neurotoxic effects of metals such as copper, manganese, and iron. Management and treatment of hepatocerebral disorders include chelation therapy (Wilson's Disease) and liver transplantation among others.

Copper accumulation has been responsible for many dysfunctions in liver, kidney and eye diseases. Excess copper is actually deposited throughout the corneas in Wilson's disease. Renal tubular dysfunction, with consequent hypercalciuria and hyperphosphaturia may induce nephroclacinosis. As pharmacological management penicillamine and trietine are being used. There have been some long term effects for using these medication and that has been discussed in the prior art (Pfeiffer 2007). There is a need for a development of new copper chelator and an anticancer metallodrug with improved specificity and decreased toxic side effects.

### SUMMARY OF DISCLOSURE

In an embodiment a compound of formula 1A is disclosed:

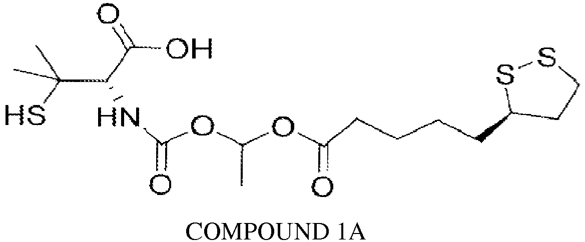

Disclosed is a compound of formula 2 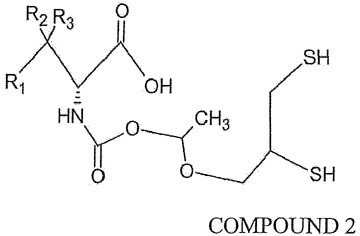 wherein R¹, R² and R³ represents, hydrogen, methyl, ethyl or thiol and R⁴ represents (RS)-2,3-disulfanylpropan-1-ol.

Further disclosed is a compound of formula 3 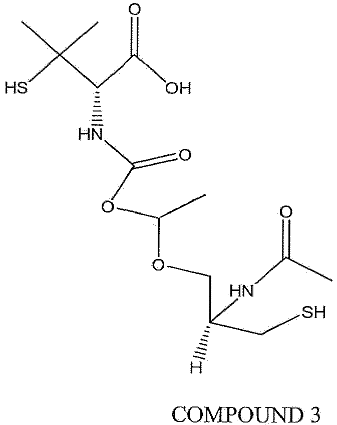 wherein R¹, R² and R³ represents, hydrogen, methyl, ethyl or thiol and R⁴ represents (R)-2-acetamido-3-sulfanylpropanoic acid.

Furthermore, this disclosure provides a composition comprising:
a) R-(+)-lipoic acid or Thioctic acid;
b) Zinc acetate (or) Triethylene tetramine; and
c) a compound of Formula 1 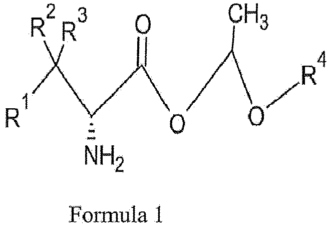 wherein,
   R¹, R², and R³ each independently represents hydrogen, thiol, alkyl, alkyl thiol, acetyl thiol, disulfide, acyl, acylalkyl, alkenyl, alkylthioalkyl, alkynyl, alkoxyaryl, alkoxyalkyl, aryl, aralkyl, aryloxyalkyl, arylthioalkyl, cycloalkyl, ether, ester, heteroaryl, heterocyclyl, lower alkyl, sulfone, sulfoxide, or hydroxyalkyl; and
   R⁴ represents at least one of a residue of guanidine, a residue of hydrazine, an acid, a residue of pyruvic acid, a residue of oxaloacetic acid, a residue of tocopherol, a residue of ascorbic acid, a residue of thiamine, thioctic acid, a residue of thioctic acid, a residue of acetyl cysteine, a residue of alpha-keto glutaric acid, a residue of dimercaprol, a residue of an NO donor, a residue of glutathione and an analog of any one of the foregoing.

It is further disclosed as a pharmaceutically acceptable composition, a pharmaceutically acceptable salt for example, but not limited to, tartrate, esylate, mesylate, sulfate, hydrate and hydrochloride salt of formula 1 comprising:
a) R-(+)-lipoic acid (or) Acetylcysteine (or) Dimercaprol;
b) Zinc acetate (or) Triethylene tetramine; and
c) a compound of Formula 1 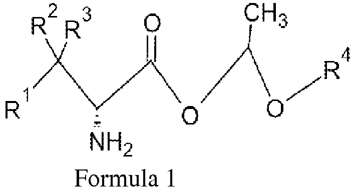
Wherein, R¹, R², and R³ each independently represents hydrogen, thiol, alkyl, alkyl thiol, acetyl thiol, disulfide, acyl, acylalkyl, alkenyl, alkylthioalkyl, alkynyl, alkoxyaryl, alkoxyalkyl, aryl, aralkyl, aryloxyalkyl, arylthioalkyl, cycloalkyl, ether, ester, heteroaryl, heterocyclyl, lower alkyl, sulfone, sulfoxide, or hydroxyalkyl; and R⁴ represents at least one of a residue of guanidine, a residue of hydrazine, an acid, a residue of pyruvic acid, a residue of oxaloacetic acid, a residue of tocopherol, a residue of ascorbic acid, a residue of thiamine, thioctic acid, a residue of thioctic acid, a residue of acetyl cysteine, a residue of alpha-keto glutaric acid, a residue of dimercaprol, a residue of an NO donor, a residue of glutathione, (RS)-2, 3-disulfanylpropan-1-ol, (R)-2-acetamido-3-sulfanylpropanoic acid and an analog of any one of the foregoing.

In one embodiment the therapeutically effective amount may be rendered, but not limited to, as an injection. Other embodiments may include peroral, topical, transmucosal, inhalation, targeted delivery and sustained release formulations. The topical application may be an ophthalmic drug used as drops, targeted delivery may be injection to the organ and peroral may be syrup, tablet or capsule.

Herein, the application additionally provides kits comprising the pharmaceutical compositions described herein. The kits may further comprise instructions for use in the treatment of diseases related to copper retention, hepatic disorders or its related complications.

Furthermore, herein is provided a kit comprising a first composition and a second composition, wherein a) the first composition is R-(+)-lipoic acid or Acetylcysteine or Dimercaprol; b) the second composition is a combination of Compound 1A and/or Compound 2 and/or Compound 3, wherein compositions not comprising compound 1A are not according to the invention, and c) the third composition is triethylene tetramine (or) Zinc acetate or Ammonium tetrathiomolybdate:

In another embodiment, R- lipoic acid, Dimercaprol, Zinc acetate, Ammonium tetrathiomolybdate or triethylene tetramine is combined with at least a pharmaceutically acceptable salt of the compound 1A.

The compound, composition, formulation, method of synthesis, and treatment disclosed herein may be implemented in any means for achieving various aspects, and may be executed in a form suitable for the mammal. Other features will be apparent from the accompanying detailed description that follows.

### BRIEF DESCRIPTION OF DRAWINGS

Figure 1 shows a first method of synthesis of compound representing compound 1A.
Figure 2 shows a second method of synthesis of compound represented by compound 2.
Figure 3 shows a second method of synthesis of compound represented by compound 3.

### DETAILED DESCRIPTION

In the present disclosure metal chelating compounds, compositions, formulations and their use are disclosed. The compounds comprise derivatives of formula 1. Furthermore, the composition of various compounds comprise of R- lipoic acid, Dimercaprol, Zinc acetate, Ammonium tetrathiomolybdate or triethylene tetramine is combined with a pharmaceutically acceptable salt of the compounds derived from formula 1. Also disclosed are methods of making the formula 1 into different compounds are disclosed.

The compound may also comprise of tartrate, esylate, mesylate, sulfate salts and hydrate salt of formula 1. Herein the application also provides a kit comprising any of the pharmaceutical compositions disclosed herein. The kit may comprise instructions for use in the treatment of diseases associated to copper toxicity, hepatic disorders or related complications.

### Definitions

As used herein, the following terms and phrases shall have the meanings set forth below. Unless defined otherwise, all technical and scientific terms used herein have the same meaning as commonly understood to one of ordinary skill in the art.

The term "alkyl" as used herein refers to a saturated linear or branched-chain monovalent hydrocarbon radical of one to twelve carbon atoms. Examples of alkyl groups include, but are not limited to, methyl (Me, -CH₃), ethyl (Et, -CH₂CH₃), 1 -propyl (n-Pr, n- propyl, -CH₂CH₂CH₃), 2-propyl (i-Pr, i-propyl, -CH(CH₃)₂), 1 -butyl (n-Bu, n-butyl, - CH₂CH₂CH₂CH₃), 2-methyl-l -propyl (i-Bu, i-butyl, -CH₂CH(CH₃)₂), 2-butyl (s-Bu, s-butyl, - CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2- methyl-2-butyl (-C(CH₃)₂CH₂CH₃), 3-methyl-2-butyl (-CH(CH₃)CH(CH₃)₂), 3 -methyl- 1 -butyl (-CH₂CH₂CH(CH₃)₂), 2-methyl-l -butyl (-CH₂CH(CH₃)CH₂CH₃), 1-hexyl (- CH₂CH₂CH₂CH₂CH₂CH₃), 2-hexyl (-CH(CH₃)CH₂CH₂CH₂ CH₃), 3-hexyl (- CH(CH₂CH₃)(CH₂CH₂CH₃)), 2-methyl-2-pentyl (-C(CH₃)₂CH₂CH₂CH₃), 3-methyl-2-pentyl (- CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (- C(CH₃)₂CH(CH₃)₂), 3,3-dimethyl-2-butyl (-CH(CH₃)C(CH₃)₃, 1-heptyl, 1-octyl, and the like. The term "alkenyl" refers to linear or branched-chain monovalent hydrocarbon radical of two to twelve carbon atoms with at least one site of unsaturation, i.e., a carbon- carbon, sp double bond, wherein the alkenyl radical includes radicals having "cis" and "trans" orientations, or alternatively, "E" and "Z" orientations. Examples include, but are not limited to, ethylenyl or vinyl (-CH=CH₂), allyl (-CH₂CH=CH₂), and the like. The term "alkynyl" refers to a linear or branched monovalent hydrocarbon radical of two to twelve carbon atoms with at least one site of unsaturation, i.e., a carbon- carbon, sp triple bond. Examples include, but are not limited to, ethynyl (-C≡CH), propynyl (propargyl, - CH₂C≡CH), and the like.

Moreover, the term "alkyl" (or "lower alkyl") as used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. Such substituents, if not otherwise specified, may include, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain may themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls may be further substituted with alkyls, alkenyls, alkoxys, alkylthios, aminoalkyls, carbonyl-substituted alkyls, -CF₃, -CN, and the like.

The term "acyl" is art-recognized and refers to a group represented by the general formula hydrocarbylC(O)-, preferably alkylC(O)-.

"Aryl" means a monocyclic or polycyclic ring assembly wherein each ring is aromatic or when fused with one or more rings forms an aromatic ring assembly. If one or more ring atoms is not carbon (e.g., N, S), the aryl is a heteroaryl. Cₓ aryl and Cₓ-_{Y} aryl are typically used where X and Y indicate the number of carbon atoms in the ring.

The term "acylamino" is art-recognized and refers to an amino group substituted with an acyl group and may be represented, for example, by the formula hydrocarbyl C(O)NH-.

The term "acylalkyl" is art-recognized and refers to an alkyl group substituted with an acyl group and may be represented, for example, by the formula hydrocarbyl C(O)alkyl.

The term "acyloxy" is art-recognized and refers to a group represented by the general formula hydrocarbylC(O)O-, preferably alkylC(O)O-.

The term "alkoxy" refers to an alkyl group, preferably a lower alkyl group, having an oxygen attached thereto. Representative alkoxy groups include methoxy, ethoxy, propoxy, tert-butoxy and the like.

The term "alkoxyalkyl" refers to an alkyl group substituted with an alkoxy group and may be represented by the general formula alkyl-O-alkyl.

The term "alkenyl", as used herein, refers to an aliphatic group containing at least one double bond and is intended to include both "unsubstituted alkenyls" and "substituted alkenyls", the latter of which refers to alkenyl moieties having substituents replacing a hydrogen on one or more carbons of the alkenyl group. Such substituents may occur on one or more carbons that are included or not included in one or more double bonds.

Moreover, such substituents include all those contemplated for alkyl groups, as discussed below, except where stability is prohibitive. For example, substitution of alkenyl groups by one or more alkyl, carbocyclyl, aryl, heterocyclyl, or heteroaryl groups is contemplated.

The term "alkylamino", as used herein, refers to an amino group substituted with at least one alkyl group.

The term "alkylthio", as used herein, refers to a thiol group substituted with an alkyl group and may be represented by the general formula alkylS-.

The term "alkynyl", as used herein, refers to an aliphatic group containing at least one triple bond and is intended to include both "unsubstituted alkynyls" and "substituted alkynyls", the latter of which refers to alkynyl moieties having substituents replacing a hydrogen on one or more carbons of the alkynyl group. Such substituents may occur on one or more carbons that are included or not included in one or more triple bonds. Moreover, such substituents include all those contemplated for alkyl groups, as discussed above, except where stability is prohibitive. For example, substitution of alkynyl groups by one or more alkyl, carbocyclyl, aryl, heterocyclyl, or heteroaryl groups is contemplated.

The term "ether", as used herein, refers to a hydrocarbyl group linked through an oxygen to another hydrocarbyl group. Accordingly, an ether substituent of a hydrocarbyl group may be hydrocarbyl-O-. Ethers may be either symmetrical or unsymmetrical. Examples of ethers include, but are not limited to, heterocycle-O-heterocycle and aryl-O-heterocycle. Ethers include "alkoxyalkyl" groups, which may be represented by the general formula alkyl-O-alkyl.

The terms "halo" and "halogen" as used herein means halogen and includes chloro, fluoro, bromo, and iodo.

The terms "hetaralkyl" and "heteroaralkyl", as used herein, refers to an alkyl group substituted with a hetaryl group.

The term "heteroalkyl", as used herein, refers to a saturated or unsaturated chain of carbon atoms and at least one heteroatom, wherein no two heteroatoms are adjacent.

The terms "heteroaryl" and "hetaryl" include substituted or unsubstituted aromatic single ring structures, preferably 5- to 7-membered rings, more preferably 5- to 6-membered rings, whose ring structures include at least one heteroatom, preferably one to four heteroatoms, more preferably one or two heteroatoms. The terms "heteroaryl" and "hetaryl" also include polycyclic ring systems having two or more cyclic rings in which two or more carbons are common to two adjoining rings wherein at least one of the rings is heteroaromatic, e.g., the other cyclic rings may be cycloalkyls, cycloalkenyls, cycloalkynyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.

The term "heteroatom" as used herein means an atom of any element other than carbon or hydrogen. Preferred heteroatoms are nitrogen, oxygen, and sulfur.

The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, e.g., the other cyclic rings may be cycloalkyls, cycloalkenyls, cycloalkynyls, aryls, heteroaryls, and/or heterocyclyls. Heterocyclyl groups include, for example, piperidine, piperazine, pyrrolidine, morpholine, lactones, lactams, and the like.

The term "heterocyclylalkyl", as used herein, refers to an alkyl group substituted with a heterocycle group.

The term "hydrocarbyl", as used herein, refers to a group that is bonded through a carbon atom that does not have a =O or =S substituent, and typically has at least one carbon-hydrogen bond and a primarily carbon backbone, but may optionally include heteroatoms. Thus, groups like methyl, ethoxyethyl, 2-pyridyl, and trifluoromethyl are considered to be hydrocarbyl for the purposes of this application, but substituents such as acetyl (which has a =O substituent on the linking carbon) and ethoxy (which is linked through oxygen, not carbon) are not. Hydrocarbyl groups include, but are not limited to aryl, heteroaryl, carbocycle, heterocycle, alkyl, alkenyl, alkynyl, and combinations thereof.

The term "hydroxyalkyl", as used herein, refers to an alkyl group substituted with a hydroxy group.

The term "ketone" is art-recognized and may be represented, for example, by the formula C (O) R₉, wherein R₉ represents a hydrocarbyl group

The term "lower" when used in conjunction with a chemical moiety, such as, acyl, acyloxy, alkyl, alkenyl, alkynyl, or alkoxy is meant to include groups where there are ten or fewer non-hydrogen atoms in the substituent, preferably six or fewer. A "lower alkyl", for example, refers to an alkyl group that contains ten or fewer carbon atoms, preferably six or fewer. Lower alkyls include methyl and ethyl. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The term "substituted" refers to moieties having substituents replacing hydrogen on one or more carbons of the backbone. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents may be one or more and the same or different for appropriate organic compounds. For purposes of this application, the heteroatoms such as nitrogen may have hydrogen substituents and/or any permissible substituents of organic compounds described herein which satisfy the valences of the heteroatoms. Substituents may include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain may themselves be substituted, if appropriate.

Unless specifically stated as "unsubstituted," references to chemical moieties herein are understood to include substituted variants. For example, reference to an "aryl" group or moiety implicitly includes both substituted and unsubstituted variants.

"Substituted or unsubstituted" means that a given moiety may consist of only hydrogen substituents through available valencies (unsubstituted) or may further comprise one or more non-hydrogen substituents through available valencies (substituted) that are not otherwise specified by the name of the given moiety. For example, isopropyl is an example of an ethylene moiety that is substituted by -CH₃. In general, a non- hydrogen substituent may be any substituent that may be bound to an atom of the given moiety that is specified to be substituted. Examples of substituents include, but are not limited to, aldehyde, alicyclic, aliphatic, (C₁₋₁₀) alkyl, alkylene, alkylidene, amide, amino, aminoalkyl, aromatic, aryl, bicycloalkyl, bicycloaryl, carbamoyl, carbocyclyl, carboxyl, carbonyl group, cycloalkyl, cycloalkylene, ester, halo, heterobicycloalkyl, heterocycloalkylene, heteroaryl, heterobicycloaryl, heterocycloalkyl, oxo, hydroxy, iminoketone, ketone, nitro, oxaalkyl and oxoalkyl moieties, each of which may optionally also be substituted or unsubstituted. In one particular embodiment, examples of substituents include, but are not limited to, hydrogen, halo, nitro, cyano, thio, oxy, hydroxy, carbonyloxy, (C₁₋₁₀) alkoxy, (C₄-₁₂) aryloxy, hetero (C₁₋₁₀)aryloxy, carbonyl, oxycarbonyl, aminocarbonyl, amino, (C₁₋₁₀) alkylamino, sulfonamido, imino, sulfonyl, sulfinyl, (C1-10)alkyl, halo (C1-10) alkyl, hydroxy (C1-10) alkyl, carbonyl (C1-10)alkyl, thiocarbonyl (C1-10)alkyl, sulfonyl (C1-10) alkyl, sulfinyl (C1-10) alkyl, (C1-10)azaalkyl, imino (C1-10) alkyl, (C₃- ₁₂) cycloalkyl (C1-₅) alkyl, hetero (C3-12) cycloalkyl (C1-10)alkyl, aryl (C1-10)alkyl, hetero (C1-10) aryl (C1-5) alkyl, (C₉- ₁₂) bicycloaryl (Ci_s) alkyl, hetero (Ce-_{I2}) bicycloaryl (C1_5) alkyl, (C3-12) cycloalkyl, hetero (C3-12) cycloalkyl, (C9-12) bicycloalkyl, hetero (C₃-_{I2}) bicycloalkyl, (C₄-_{I2}) aryl, hetero (C1-10) aryl, (C₉- ₁₂) bicycloaryl and hetero (C₄-₁₂) bicycloaryl. In addition, the substituent is itself optionally substituted by a further substituent. In one particular embodiment, examples of the further substituent include, but are not limited to, hydrogen, halo, nitro, cyano, thio, oxy, hydroxy, carbonyloxy, (C1-10)alkoxy, (C₄-I₂) aryloxy, hetero (CI-10) aryloxy, carbonyl, oxycarbonyl, aminocarbonyl, amino, (CI-10) alkylamino, sulfonamido, imino, sulfonyl, sulfinyl, (C1-10) alkyl, halo (C1-10)alkyl, hydroxy (C1-10) alkyl, carbonyl (C1-10) alkyl, thiocarbonyl (C1-10) alkyl, sulfonyl (C1-10) alkyl, sulfinyl (C1-10)alkyl, (CI-10) azaalkyl, imino (CI-10) alkyl, (C₃-₁₂) cycloalkyl (C₁- ₅) alkyl, hetero (C3-12) cycloalkyl (C1-10) alkyl, aryl (C_{1_10}) alkyl, hetero (Ci-io) aryl (Ci_₅) alkyl, (C₉-I₂) bicycloaryl (C₁-₅) alkyl, hetero (C₈-₁₂) bicycloaryl (Ci_s) alkyl, (C3-12) cycloalkyl, hetero (C₃_ 12) cycloalkyl, (C₉-₁₂) bicycloalkyl, hetero (C₃-₁₂) bicycloalkyl, (C₄-₁₂) aryl, hetero (C1-10) aryl, (C₉-₁₂) bicycloaryl and hetero (C₄- ₁₂) bicycloaryl.

The compounds of the present compound of formula 1 may be present in the form of pharmaceutically acceptable salts. The compounds of the present disclosure may also be present in the form of pharmaceutically acceptable esters (i.e., the methyl and ethyl esters of the acids of formula I to be used as prodrugs). The compounds of the present disclosure may also be solvated, i.e. hydrated. The solvation may be effected in the course of the manufacturing process or may take place i.e. as a consequence of hygroscopic properties of an initially anhydrous compound of formula I (hydration).

Compounds that have the same molecular formula but differ in the nature or sequence of bonding of their atoms or the arrangement of their atoms in space are termed "isomers." Isomers that differ in the arrangement of their atoms in space are termed "stereoisomers." Diastereomers are stereoisomers with opposite configuration at one or more chiral centers which are not enantiomers. Stereoisomers bearing one or more asymmetric centers that are non- superimposable mirror images of each other are termed "enantiomers." When a compound has an asymmetric center, for example, if a carbon atom is bonded to four different groups, a pair of enantiomers is possible. An enantiomer may be characterized by the absolute configuration of its asymmetric center or centers and is described by the R- and S-sequencing rules of Cahn, Ingold and Prelog, or by the manner in which the molecule rotates the plane of polarized light and designated as dextrorotatory or levorotatory (i.e., as (+) or (-)-isomers respectively). A chiral compound may exist as either individual enantiomer or as a mixture thereof. A mixture containing equal proportions of the enantiomers is called a "racemic mixture".

The term "sulfate" is art-recognized and refers to the group OSO₃H, or a pharmaceutically acceptable salt thereof. A sulfate of compound of formula 1 or crystal thereof may be a hydrate. The number of the combined water can be controlled by varying the condition of recrystallization or drying. The salt form may be hydrochloride salt as well.

The term "polymorph" as used herein is art-recognized and refers to one crystal structure of a given compound.

"Residue" is an art-recognized term that refers to a portion of a molecule. For instance, a residue of thioctic acid may be: dihydrolipoic acid, bisnorlipoic acid, tetranorlipoic acid, 6,8-bismethylmercapto-octanoic acid, 4,6-bismethylmercapto-hexanoic acid, 2,4-bismethylmeracapto-butanoic acid, 4,6-bismethylmercapto-hexanoic acid.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if it is administered after manifestation of the unwanted condition, the treatment is therapeutic, (i.e., it is intended to diminish, ameliorate, or stabilize the existing unwanted condition or side effects thereof).

The term "solvate" as used herein, refers to a compound formed by solvation (e.g., a compound formed by the combination of solvent molecules with molecules or ions of the solute).

This application also discloses a pharmaceutical composition comprising a pharmaceutically acceptable carrier and the composition of thioctic acid or a residue of thioctic acid, dimercaprol or acetylcyteine and salts of compound 1A This application further discloses a pharmaceutical composition comprising a pharmaceutically acceptable carrier and (a) lipoic acid or residue of lipoate and (b) a compound of Formula I (c) dimercaprol or acetylcysteine or zinc acetate or ammonium thiomolybdate. The pharmaceutical composition may be formulated for systemic or topical administration. The pharmaceutical composition may be formulated for oral administration, injection, subdermal administration, or transdermal administration. The pharmaceutical composition may further comprise at least one of a pharmaceutically acceptable stabilizer, diluent, surfactant, filler, binder, and lubrimayt.

Additionally, the optimal concentration and/or quantities or amounts of any particular compound 1A or composition may be adjusted to accommodate variations in the treatment parameters. Such treatment parameters include the clinical use to which the preparation is put, e.g., the site treated, the type of patient, e.g., human or non-human, adult or child, and the nature of the disease or condition.

Wilson's disease (WD) is an autosomal recessive disorder of the copper metabolism leading to the accumulation of this metal in different organs and tissues. Hepatic and neurological symptoms are the main clinical features of the disease. Copper-associated diseases are increasingly being reported in both man and animals. Copper also has a role in fatal, non-Wilson's liver diseases affecting young children with a genetic abnormality of copper metabolism. Excess accumulation of copper also occurs as a consequence of chronic liver diseases such as primary biliary cirrhosis, and chronic hepatitis in mammal such as humans and animals.

In certain embodiments, the compound 1A and compositions herein may be used to treat one or more copper toxicity related diseases or complications. Complications include Hepatic (cirrhosis, chronic active hepatitis, fulminant hepatic failure), Neurologic (bradykinesia, rigidity, tremor, ataxia, dyskinesia, dysarthria, seizures), Psychiatric (behavioral disturbances, cognitive impairment, psychosis), Orthalmologic (kayser-Fleischer rings, sunflow cataracts), Hematologic (haemolysis, coagulopathy), Renal (renal tubular defects, diminished glomerular filtration, nephrolithiasis), Cardiovascular (cardiomyopathy, arrhythmias, conduction disturbances, autonomic dysfunction), Musculoskeletal (osteomalacia, osteoporosis, degenerative joint diseases), Gastrointestinal (cholelithiasis, pancreatitis, bacterial peritonitis), Endocrinologic (amenorrhoea, spontaneous abortion, delayed puberty, gynecomastia), Dermatologic (hyperpigmentation, amaythosis nigrimays).

The compositions may be administered alone or in combination with pharmaceutically acceptable carriers, vehicles or diluents, in either single or multiple doses and with other drugs for example a pain killer, but not limited to it.. Suitable pharmaceutical carriers, vehicles and diluents include inert solid diluents or fillers, sterile aqueous solutions and various organic solvents. The pharmaceutical compositions formed by combining the compositions and the pharmaceutically acceptable carriers, vehicles or diluents are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as L-arginine, sodium citrate, calcium carbonate and calcium phosphate may be employed along with various disintegrates such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Appropriate materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

In addition to the active or therapeutic ingredients, tablets may contain a number of inert materials known as excipients. They may be classified according to the role they play in the final tablet. The primary composition may include one or more of a filler, binder, lubricant and glidant. Other excipients which give physical characteristics to the finished tablet are coloring agents, and flavors (especially in the case of chewable tablets). Without excipients most drugs and pharmaceutical ingredients cannot be directly-compressed into tablets. This is primarily due to the poor flow and cohesive properties of most drugs. Typically, excipients are added to a formulation to impart good flow and compression characteristics to the material being compressed. Such properties are imparted through pretreatment steps, such as wet granulation, slugging, spray drying spheronization or crystallization.

Lubricants are typically added to prevent the tableting materials from sticking to punches, minimize friction during tablet compression, and allow for removal of the compressed tablet from the die. Such lubricants are commonly included in the final tablet mix in amounts usually of about 1% by weight.

Other desirable characteristics of excipients include the following: high-compressibility to allow strong tablets to be made at low compression forces; impart cohesive qualities to the powdered material; acceptable rate of disintegration; good flow properties that can improve the flow of other excipients in the formula; and cohesiveness (to prevent tablet from crumbling during processing, shipping and handling).

There are at least three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

One formulation comprises the following: a compound of Formula I, and a binder. Examples of pharmaceutically acceptable binders include, but are not limited to, starches; celluloses and derivatives thereof, e.g., microcrystalline cellulose, hydroxypropyl cellulose hydroxylethyl cellulose and hydroxylpropylmethyl cellulose; sucrose; dextrose; corn syrup; polysaccharides; and gelatin. The binder, e.g., may be present in an amount from about 1 % to about 40% by weight of the composition such as 1 % to 30% or 1 % to 25% or 1 % to 20%.

Optionally, one, two, three or more diluents can be added to the formulations disclosed herein. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition. In certain embodiments, diluents are microcrystalline cellulose which is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. The microcrystalline cellulose may be present in a tablet formulation in an amount of from about 25% to about 70% by weight. Another appropriate range of this material is from about 30% to about 35% by weight; yet another appropriate range of from about 30% to about 32% by weight. Another diluent is lactose. The lactose may be ground to have an average particle size of between about 50 µm and about 500 µm prior to formulating. The lactose may be present in the tablet formulation in an amount of from about 5% to about 40% by weight, and can be from about 18% to about 35% by weight, for example, can be from about 20% to about 25% by weight.

Optionally one, two, three or more disintegrants can be added to the formulations described herein. Examples of pharmaceutically acceptable disintegrants include, but are not limited to, starches; clays; celluloses; alginates; gums; cross-linked polymers, e.g., cross- linked polyvinyl pyrrolidone, cross-linked calcium carboxymethylcellulose and cross-linked sodium carboxymethylcellulose; soy polysaccharides; and guar gum. The disintegrant, e.g., may be present in an amount from about 2% to about 20%, e.g., from about 5% to about 10%, e.g., about 7% about by weight of the composition. A disintegrant is also an optional but useful component of the tablet formulation. Disintegrants are included to ensure that the tablet has an acceptable rate of disintegration. Typical disintegrants include starch derivatives and salts of carboxymethylcellulose. Sodium starch glycolate is one appropriate disintegrant for this formulation. In certain embodiments, the disintegrant is present in the tablet formulation in an amount of from about 0% to about 10% by weight, and can be from about 1% to about 4% by weight, for instance from about 1.5% to about 2.5% by weight.

Optionally one, two, three or more lubricants can be added to the formulations disclosed herein. Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant, e.g., may be present in an amount from about 0.1% to about 5% by weight of the composition; whereas, the glidant, e.g., may be present in an amount from about 0.1% to about 10% by weight. Lubricants are typically added to prevent the tableting materials from sticking to punches, minimize friction during tablet compression and allow for removal of the compressed tablet from the die. Such lubricants are commonly included in the final tablet mix in amounts usually less than 1% by weight. The lubricant component may be hydrophobic or hydrophilic. Examples of such lubricants include stearic acid, talc and magnesium stearate. Magnesium stearate reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. One lubricant, magnesium stearate may also be employed in the formulation. In some aspects, the lubricant is present in the tablet formulation in an amount of from about 0.25% to about 6%; also appropriate is a level of about 0.5% to about 4% by weight; and from about 0.1% to about 2% by weight. Other possible lubricants include talc, polyethylene glycol, silica and hardened vegetable oils. In an optional embodiment, the lubricant is not present in the formulation, but is sprayed onto the dies or the punches rather than being added directly to the formulation.

Other conventional solid fillers or carriers, such as, cornstarch, calcium phosphate, calcium sulfate, calcium stearate, magnesium stearate, stearic acid, glyceryl mono- and distearate, sorbitol, mannitol, gelatin, natural or synthetic gums, such as carboxymethyl cellulose, methyl cellulose, alginate, dextran, acacia gum, karaya gum, locust bean gum, tragacanth and the like, diluents, binders, lubricants, disintegrators, coloring and flavoring agents could optionally be employed.

Additional examples of useful excipients which can optionally be added to the composition are described in the Handbook of Pharmaceutical Excipients, 3rd edition, Edited by A.H.Kibbe, Published by: American Pharmaceutical Association, Washington DC, ISBN: 0-917330-96-X, or Handbook of Pharmaceutical Excipients (4th edition), Edited by Raymond C Rowe - Publisher: Science and Practice.

The formulations, for instance tablets, may contain e.g. 3 to 800, or 20 to 600, e.g. 50, 250, 300, or 400, mg of compound 1A and compositions disclosed herein, for instance, compound 1A or salts of compound 1A or a composition comprising (a) lipoic acid or residue of lipoate and (b) compound 1A and, (c) dimercaprol or acetylcysteine or zinc acetate or ammonium thiomolbdate.

The subject compositions may be administered once, or may be divided into a number of smaller doses to be administered at varying intervals of time, depending in part on the release rate of the compositions and the desired dosage.

Ophthalmic formulations, eye ointments, powders, solutions and the like, are also contemplated as being within the scope of the disclosures herein. Examples of suitable aqueous and non-aqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

### METHODS OF SYNTHESIS

### EXAMPLE SYNTHESIS 1:

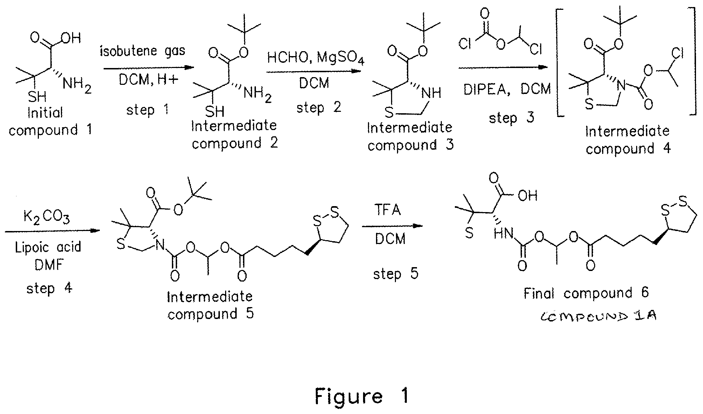

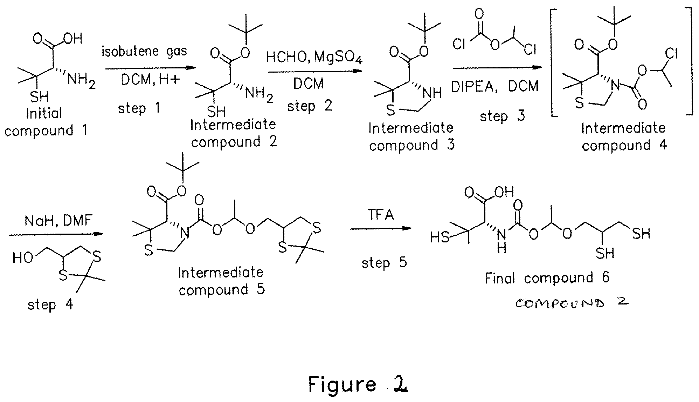

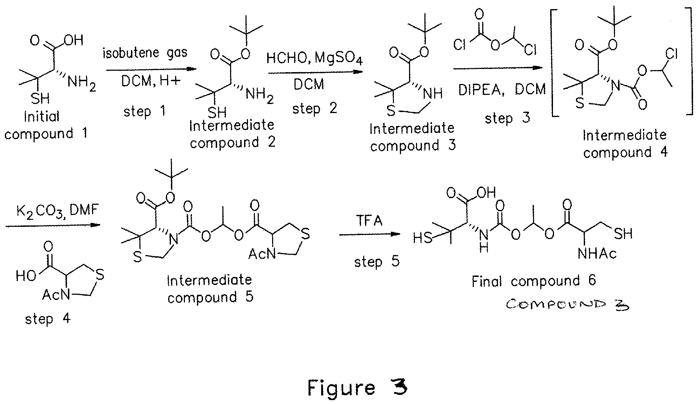

Figures 1, 2 and 3 show a five step synthesis process for the composition of formula 1 for various compounds such as compound 1A, 2 and 3. Compositions with compounds 2 and 3 are reference examples.
Step 1for compound 1A, 2 and 3: (2S)-2-amino-3-methyl-3-sulfanyl-butanoic acid (initial compound 1) or D-(-)- Pencillamine and Dichloromethane (DCM) were mixed together as a reaction mixture in a pressure bottle containing a magnetic stirrer. The pressure bottle containing the reaction mixture (intermediate compound 1) was securely closed with a rubber septum. The pressure bottle containing the reaction mixture was further cooled in 2-isoproponol / dry ice at 7-8°C in the dry ice bath. Condensed isobutylene was transferred to the pressure bottle, using a cannula, followed by adding a few drops of sulfuric acid to the reaction mixture. The addition of isobutylene was continued for a period of 2 hours. Stirring of the reaction mixture was continued at room temperature for an additional 16 hours. The pressure bottle was kept in *i*-PrOH/dry ice bath and rubber septum was carefully removed. The reaction mixture was allowed to degas fully by stirring for several minutes. Saturated aqueous NaHCO3 was added to the reaction mixture, and the resultant reaction mixture was stirred for 2 hours at room temperature. The pH of the aqueous layer was measured and recorded as pH 8. Water was added for the removal of the emulsion that was formed during the neutralization step. The aqueous layer was treated using with DCM and then extracted. The entire DCM extracts were pooled together. The pooled DCM extracts were washed with saturated aqueous NaHCO3, water, and saturated aqueous NaCl solution. The resultant organic layer was dried in under MgSO4 atmosphere, concentrated and filtered under reduced pressure to yield intermediate compound 2.
Step 2 for compound 1A, 2 and 3: The condensation of amino thiol with paraformaldehyde in ethanol at room temperature for 30 minutes yielded thiazolidine derivative as intermediate compound 3.
Step 3 for compound 1A, 2 and 3: Thiazolidine derivative intermediate compound 3 was treated with 1.0 equivalents of 1-chloroethylchloroformate in presence of 1.5 equivalents of N,N-Diisopropylethylamine (DIPEA) in anhydrous dimercaprol at 0°C. The reaction mixture was allowed to stir for 30 min at 0°C and yielded intermediate compound 4. On completion of the reaction the quality was monitored and recorded by performing thin layer chromatography (TLC). Based on the observation if the quality was satisfactory the intermediate compound 4 of step 3 was then directly used for the next step, without any further purification process.
Step 4 for compound 1A: Potassium salt of Lipoic acid was obtained from reacting lipoic acid and anhydrous K₂CO₃ under dry Dimethylformaldehyde at 0°C. This reaction mixture of step 3 was added slowly into the above solution and then the crude reaction mixture was allowed to stir for 16 h at room temperature. Reaction was monitored by TLC. The crude reaction mixture was then vacuum distilled and fractionated using water and dichloromethane. The combined aqueous and organic layers were washed with brine solution, dried over anhydrous Na₂SO₄ and evaporated under reduced pressure. The crude reaction mixture was purified by column chromatography over 100-200 mesh silica gel to yield Lipoic acid derivative intermediate compound 5.
Step 4 for compound 2: Intermediate compound 4 was added slowly to the solution of (2, 2-dimethyl-1, 3-dithiolan-4-yl) methanol sodium salt in dry dimethylformamide (DMF) at 0°C to make reaction mixture 3. The reaction mixture 3 was stirred for 16 hour at room temperature. The reaction mixture 3 was dried using evaporation technique. The dried reaction mixture was divided and then washed with water and DCM. The combined organic layers were washed with brine solution, dried over anhydrous Na₂SO₄ and then evaporated under reduced pressure. The resultant crude was purified by column chromatography over 100-200 mesh silica gel to yield compound 5.
Step 4 for compound 3: Intermediate compound 4 was added slowly to the solution of 3-acetylthiazolidine-4-carboxylic acid potassium salt in dry dimethylformamide (DMF) at 0°C to make reaction mixture 3. The reaction mixture 3 was stirred for 16 hour at room temperature. The reaction mixture 3 was dried using evaporation technique. The dried reaction mixture was divided and then washed with water and DCM. The combined organic layers were washed with brine solution, dried over anhydrous Na₂SO₄ and then evaporated under reduced pressure. The resultant crude was purified by column chromatography over 100-200 mesh silica gel to yield compound 5.
Step 5 for compound 1A: Intermediate compound 5 obtained in the previous step 4 was treated with 25% trifluoracetic acid dissolved in DCM to hydrolyse the tert-butyl ester with the thiazolidine group of intermediate compound 5. This reaction yielded the final compound 6.
Step 5 for compound 2: The final step is hydrolysis of tert-butyl ester, acetonide and thiazolidine group of intermediate compound 5. Intermediate compound 5 is treated with 25% TFA dissolved in DCM to produce final compound 6. In one embodiment, the Tert-butyl ester can be prepared using 1-ethyl-3-(3'-dimethylaminopropyl) carbodiimide (EDCI) coupling conditions. It may be prepared by reacting D-(-)-Pencillamine with t-butanol using EDCI coupling conditions. In another embodiment, first one may protect aminothiol of D-(-)-Penicillamine and then react with Boc anhydride and 4-(N,N-dimethylamino) pyridine (DMAP) dissolved in DCM. In another embodiment, the amino acid may be converted to tert-butlyester by reacting the amino acid with t-butanol, magnesium sulfate and sulfuric acid mixed with DCM.
Step 5 for compound 3: The final step is hydrolysis of tert-butyl ester, acetonide and thiazolidine group of intermediate compound 5. Intermediate compound 5 is treated with 25% TFA dissolved in DCM to produce final compound 6. In one embodiment, the Tert-butyl ester can be prepared using 1-ethyl-3-(3'-dimethylaminopropyl) carbodiimide (EDCI) coupling conditions. It may be prepared by reacting (2S)-2-amino-3-methyl-3-sulfanyl-butanoic acid with t-butanol using EDCI coupling conditions. In another embodiment, first one may protect aminothiol of (2*S*)-2-amino-3-methyl-3-sulfanyl-butanoic acid and then react with SBoc anhydride and 4-(N,N- dimethylamino) pyridine (DMAP) dissolved in DCM. In another embodiment, the amino acid may be converted to tert-butlyester by reacting the amino acid with t-butanol, magnesium sulfate and sulfuric acid mixed with DCM.

### RESULTS OF SYNTHESIS :

### Compound 1A:

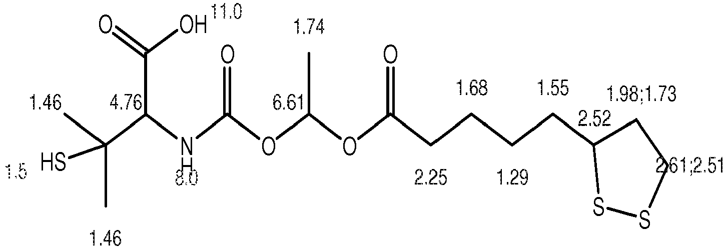

M.F : C16H27NO6S3, Mol. Wt.: 426

**Table 1: CHN Analysis**

| Atom | Intensity |
|---|---|
| C | 45.15 |
| H | 6.39 |
| N | 3.29 |
| O | 22.56 |
| S | 22.60 |

**Table 2: H NMR Analysis**

| δ | Protons | Group |
|---|---|---|
| 1.46 | 6H | 2xCH₃ |
| 1.29,1.55,1.68,1.98,2.25 | 10H | 5xCH₂ |
| 1.74 | 3H | CH₃ |
| 2.51-2.61 | 3H | SCH, SCH₂ |
| 4.76 | 2H | SCH₂N |
| 4.68 | 1H | CH |
| 6.61 | 1H | OCHO |

### Compound 2:

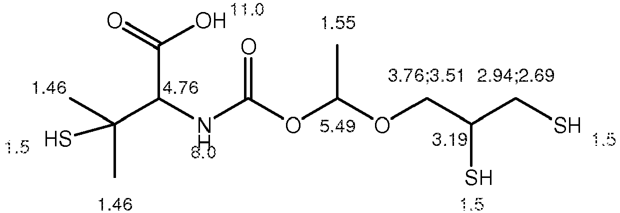

M.F : C11H21NO5S3, Mol. Wt.: 343

**Table 3-CHN Analysis**

| Atom | Intensity |
|---|---|
| C | 38.46 |
| H | 6.16 |
| N | 4.08 |
| O | 23.29 |
| S | 28.01 |

**Table 4 -H NMR Analysis**

| δ | Protons | Group |
|---|---|---|
| 1.46 | 6H | 2xCH₃ |
| 1.55 | 3H | CH₃ |
| 2.69-3.19 | 3H | SCH, SCH₂ |
| 4.76 | 1H | CHN |
| 5.49 | 1H | OCHO |

### Compound 3:

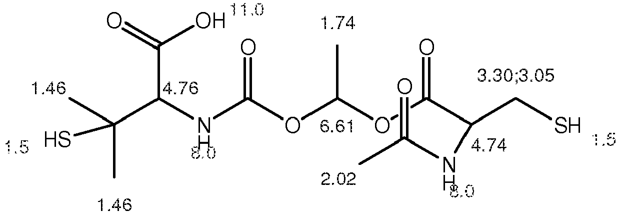

M.F : C13H22N2O7S2, Mol. Wt.: 382

**Table 5 -CHN Analysis**

| Atom | Intensity |
|---|---|
| C | 40.83 |
| H | 5.80 |
| N | 7.32 |
| O | 29.28 |
| S | 16.77 |

**Table 6-H NMR Analysis**

| δ | Protons | Group |
|---|---|---|
| 1.46 | 6H | 2xCH₃ |
| 1.74 | 3H | CH₃ |
| 2.02 | 3H | NAc |
| 3.3 | 2H | SCH₂ |
| 4.74-4.76 | 2H | 2xCHN |
| 6.61 | 1H | OCHO |

In another embodiment, an effective dosage for the compound of Formula 1 is in the range of about 0.3 mg/kg/day to about 60 mg/kg/day in single or divided doses, for instance 1 mg/kg/day to about 50 mg/kg/day in single or divided doses. The compounds of Formula 1 may be administered at a dose of, for example, less than 2 mg/kg/day, 5 mg/kg/day, 10 mg/kg/day, 20 mg/kg/day, 30 mg/kg/day, or 40 mg/kg/day. Compounds of Formula 1 may also be administered to a human patient at a dose of, for example, between 50 mg and 1000 mg, between 100 mg and 800 mg, or less than 1000, 900, 800, 700, 600, 500, 400, 300, 200, or 100 mg per day. In certain embodiments, the compositions herein are administered at an amount that is less than 95%, 90%, 80%, 70%, 60%, 50%, 40%, 30%, 20%, or 10% of the compounds of formula 1 is required for the same therapeutic benefit. The sustained release tablets may have a coating that may release the drug in a slow manner.

The present disclosure provides among other things compositions and use of compounds 1A for treating Copper toxicity related diseases and complications.

### INDUSTRIAL APPLICABILITY

There are multiple applications for compound of formula 1A, composition of formula 1A with pharmaceutically acceptable additives to treat mammals suffering from hepatic diseases, more specifically genetic and abnormal accumulation of metal in the liver in general. These compositions may be used in the treatment of diseases related to copper retention and its complications in hepatic diseases.

## Claims

1. A compound of Formula 1A 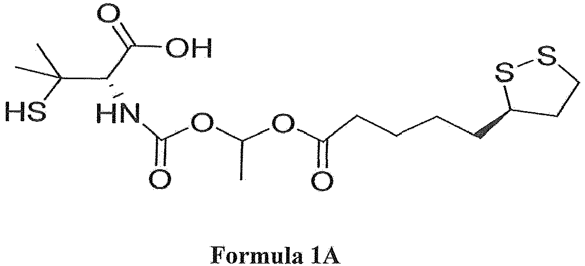 or a pharmaceutically acceptable salt thereof.

2. The compound of claim 1, wherein the pharmaceutically acceptable salt of the compound of Formula 1A is at least one of a tartrate, esylate, mesylate, sulfate, hydrate and hydrochloride salt.

3. A pharmaceutical composition comprising a compound of claims 1 or 2, and at least one of R-(+)-lipoic acid, acetylcysteine and dimercaprol, and at least one of zinc acetate and triethylene tetramine.

4. The compound of claims 1 or 2, or the composition of claim 3, for use in the treatment of a mammal with a hepatic disorder.

5. The compound for the use of claim 4, or the composition for the use of claim 4, wherein the hepatic disorder is related to copper toxicity.

6. The compound for the use of claims 4 or 5, or the composition for the use of claims 4 or 5, wherein the hepatic disorder is Wilson's disease.

7. The compound for the use of claims 4 to 6, or the composition for the use of claims 4 to 6, wherein the compound or the composition is administered in the form of at least one of a peroral, topical, transmucosal, inhalation, targeted delivery and sustained release formulation.

8. The compound for the use of claim 7, or the composition for the use of claim 7, wherein the formulation is at least one of an ophthalmic solution, injection and tablet.

## Patentansprüche

1. Verbindung der Formel 1A 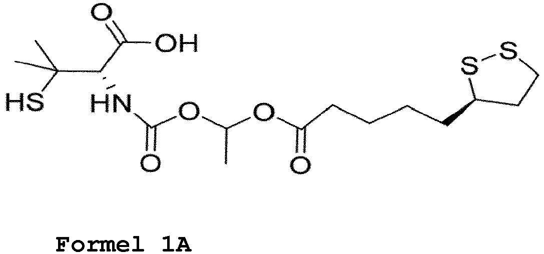 oder ein pharmazeutisch unbedenkliches Salz davon.

2. Verbindung nach Anspruch 1, wobei das pharmazeutisch unbedenkliche Salz der Verbindung der Formel 1A mindestens eines von einem Tartrat, Esylat, Mesylat, Sulfat, Hydrat und Hydrochloridsalz ist.

3. Pharmazeutische Zusammensetzung, umfassend eine Verbindung nach den Ansprüchen 1 oder 2, und mindestens eines von R-(+)-Liponsäure, Acetylcystein und Dimercaprol und mindestens eines von Zinkacetat und Triethylentetramin.

4. Verbindung nach den Ansprüchen 1 oder 2, oder Zusammensetzung nach Anspruch 3, zur Verwendung in der Behandlung eines Säugetiers mit einer Lebererkrankung.

5. Verbindung zur Verwendung nach Anspruch 4, oder Zusammensetzung zur Verwendung nach Anspruch 4, wobei die Lebererkrankung mit Kupfertoxizität in Zusammenhang steht.

6. Verbindung zur Verwendung nach den Ansprüchen 4 oder 5, oder Zusammensetzung zur Verwendung nach den Ansprüchen 4 oder 5, wobei die Lebererkrankung Morbus Wilson ist.

7. Verbindung zur Verwendung nach den Ansprüchen 4 bis 6, oder Zusammensetzung zur Verwendung nach den Ansprüchen 4 bis 6, wobei die Verbindung oder Zusammensetzung in Form mindestens eines von einer oralen, topischen und transmukosalen Formulierung, Inhalationsformulierung, Formulierung mit gezielter Verabreichung und Formulierung mit verzögerter Freisetzung verabreicht wird.

8. Verbindung zur Verwendung nach Anspruch 7, oder Zusammensetzung zur Verwendung nach Anspruch 7, wobei die Formulierung mindestens eines von einer ophthalmischen Lösung, Injektion und Tablette ist.

## Revendications

1. Composé de Formule 1A 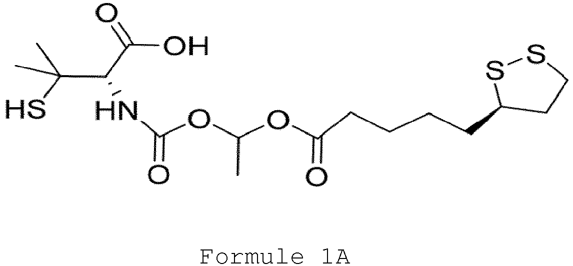 ou sel pharmaceutiquement acceptable de celui-ci.

2. Composé selon la revendication 1, où le sel pharmaceutiquement acceptable du composé de Formule 1A est au moins l'un des éléments du groupe constitué par les suivants : sel de tartrate, ésylate, mésylate, sulfate, hydrate et chlorhydrate.

3. Composition pharmaceutique comprenant un composé selon la revendication 1 ou 2, et au moins l'un des éléments du groupe constitué par acide R-(+)-lipoïque, acétylcystéine et dimercaprol, et au moins l'un des éléments du groupe constitué par acétate de zinc et triéthylènetétramine.

4. Composé selon la revendication 1 ou 2, ou composition selon la revendication 3, pour utilisation dans le traitement d'un mammifère souffrant d'un trouble hépatique.

5. Composé pour utilisation selon la revendication 4, ou composition pour utilisation selon la revendication 4, où le trouble hépatique est lié à la toxicité du cuivre.

6. Composé pour utilisation selon la revendication 4 ou 5, ou composition pour utilisation selon la revendication 4 ou 5, où le trouble hépatique est la maladie de Wilson.

7. Composé pour utilisation selon les revendications 4 à 6, ou composition pour utilisation selon les revendications 4 à 6, où le composé ou la composition sont administrés sous la forme d'au moins l'un des membres du groupe constitué par les formules perorale, topique, transmuqueuse, pour inhalation, à libération ciblée et à libération prolongée.

8. Composé pour utilisation selon la revendication 7, ou composition pour utilisation selon la revendication 7, où la formule est au moins l'un des membres du groupe constitué par une solution ophtalmique, une injection et un comprimé.
